# EUROPEAN PATENT APPLICATION

(11) **EP 4 227 683 A1**
(43) Date of publication of application: **16.08.2023**
(21) Application number: 22155895.0
(22) Date of filing: 09.02.2022
(51) Int. Cl.: G01N 33/543, G01N 33/53, G01N 33/68, G01N 33/82, G01N 33/74

(54) **BROAD SCREENING OF METABOLITES**

(71) Applicant: Dianox ApS, 2100 København Ø (DK)
(72) Inventor: CODE, Christian Sean, 5000 Odense C (DK); KONJEN, Huram, 2620 Albertslund (DK)
(74) Representative: Zacco Denmark A/S

(57) **Abstract**

A diagnostic microarray plate for detecting or quantifying multiple metabolites (e.g. hormones, vitamins, salts, sterols and/or triglycerides) from a biofluid sample of a subject. The plate comprises liposomal nanoparticles and aptamers or nanobodies specific for the metabolites of interest. A reporter material induces a colorimetric change that is visible by absorbance or fluorescence spectroscopy or a chemical that changes color through a redox reaction.

## Description

### Technical field of the invention

The present invention relates to a diagnostic microarray plate, to detect multiple metabolites from a biofluid sample of a subject.

### Background of the invention

In the medical field providing diagnosis of diseases and ailments, biofluid samples are often subjected to a range of biochemical analyses to provide data assisting in the diagnosis of the disease or ailment. Many of these analyses have traditionally been performed using different analysis methods for each metabolite that is detected or quantified.

EP2786150B1 describes a method for detection of multiple analytes.

EP1667780A1 describes a method of detecting multiple analytes.

US95/23683 describes functionalized polydiacetylene sensors.

WO11/057347 describes analyte detection using aptamers to bind to specific analytes.

There is a need in the medical field for being able to analyze biofluid samples in a single workflow to determinate the presence and/or concentrations of multiple metabolites.

It is an object of the present invention to provide diagnostic analysis materials for enabling fast analysis for a range of metabolites in a biofluid sample in a single workflow. It is also an object of the present invention to provide such diagnostic analysis materials enabling faster, cheaper and more reliable analysis results, which trickles into faster and more reliable diagnosis of disease and ailments.

### Summary of the invention

In a first aspect the present invention provides a diagnostic microarray plate having a multitude of areas or wells for identifying or quantifying multiple metabolites in a biofluid, comprising:
I. a plastic base material with liposomal nanoparticles (liposomes or lipid embedded or attached or added) on the multitude of areas or wells of said base material; and
II. at least one element is present in each of the relevant areas or wells of the plate comprising an aptamer or a nanobody that is covalently attached to said liposomal nanoparticles, wherein said aptamer or nanobody can bind to at least one metabolite potentially present in said biofluid;
III. a reporter material comprising a polymer, such as polydiacetylene, with or without a lipid that upon contact with a specific metabolite from said biofluid can generate a colorimetric change that is visible by absorbance or fluorescence spectroscopy or a chemical that changes color through a redox reaction.

In a second aspect the present invention provides the use of a diagnostic microarray plate according to the first aspect for signature pattern analysis of a biofluid sample from a single subject.

In a third aspect the present invention provides the use of a diagnostic microarray plate according to the first aspect for the detection or quantification of at least 5 hormones and vitamins selected from the group consisting of: Cortisol, Dehydroepiandrosterone (DHEA), Estradiol (17-Beta-Estradiol), Estriol, Estrone, Progesterone, Testosterone, Thyroid T4 thyroxine, Vitamin A (retinol) Binding Protein, Vitamin B1 (thiamine), Vitamin B2 (riboflavin), and Vitamin D (25-Hydroxyvitamin D).

In a fourth aspect the present invention provides the use of a diagnostic microarray plate according to the first aspect for detection or quantification wherein the shape of the well sediments the cells on the bottom surface and concentrates lipid nanoparticles on the upper surface.

### Brief description of the drawings

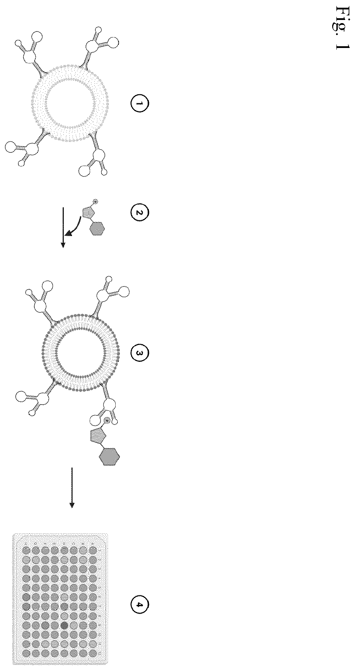

Fig. 1 Shows a lipid nanoparticle with an aptamer attached (1) and upon addition of a metabolite (2) the lipid nanoparticle changes color as a result of a reporter embedded in the lipid nanoparticle (3). Lipid nanoparticles containing different aptamers can be placed onto the plate to detect different metabolites (4).

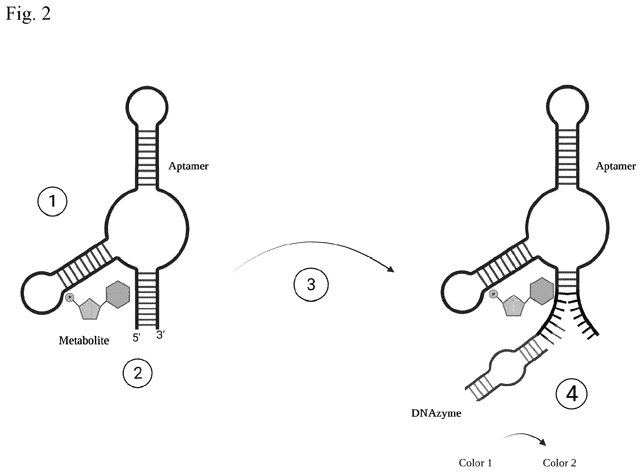

Fig. 2 Shows a second embodiment of an aptamer binding to a metabolite (1) where a complementary DNA sequence subsequently binds (3) to the partly unzipped aptamer (2), which DNA sequence is attached to a DNAzyme reporter system (4).

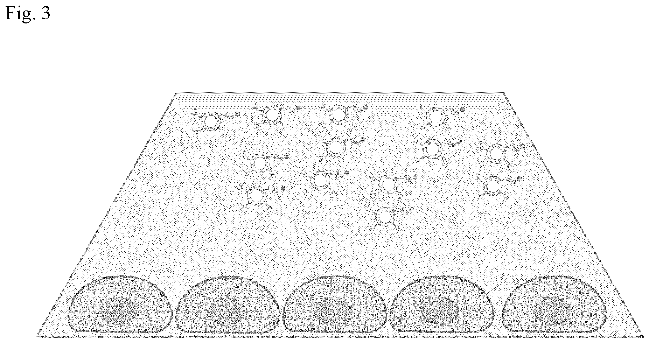

Fig. 3 Shows the chamber of a well on the diagnostic microarray plate with an isosceles trapezoid shape where the cells sediment on the bottom surface and the lipid nanoparticles concentrate on the upper surface.

### Description

A metabolite is a substance viz. (hormones, vitamins, salts, sterols and/or triglycerides) that is traceable in a biofluid, e.g. blood, saliva, urine, and other biological samples. Analysis of metabolites may require development of specific binders like aptamers or nanobodies through chemical modification that can specifically bind to the metabolite of interest. The aptamers or nanobodies can be bound to a particular reporter embedded within a liposome nanoparticle. The liposome nanoparticle can be added to a plastic plate. If the metabolite is present, the liposome nanoparticle changes its structure upon binding, which exhibits a color change.

In another embodiment, aptamers or nanobodies are directly attached to a plate. The aptamer or nanobody exhibits a structural change upon binding the metabolite allowing for a reporter element to bind. An additional enzyme or DNAzyme is added to the plate which causes a redox reaction on a chemical that exhibits a colorimetric change. The reporter from the first embodiment (the liposome with an embedded polymer chromophore) or the external oxidized chromophore can be detected with UV-Vis or fluorescent spectroscopy that can be used to quantify the specific metabolite. The binder and reporter can be put into the plate that can be used to detect multiple metabolites from the biofluid of a single subject for signature pattern analysis.

The diagnostic microarray plates of the present invention provides a novel approach for detecting multiple metabolites in e.g. a single 96 or 384-well microtiter plate.

Hence, the microarray plate may have from 2-384 different aptamers or nanobodies against specific metabolites found in biofluids which, when in contact with the metabolite, is triggered when a minimum metabolite concentration is reached. The minimum metabolite concentration in biofluids generates a change in the lipid nanoparticles detectable by absorbance or fluorescence spectroscopy at specific concentrations.

The lipid nanoparticle in which the element is covalently bonded, either directly or via an anchoring element linked to it, may have a size in the range from about 50 to about 3000 nm, in which the particle is based on a chromophoric polymer, and in which the element, and/or the anchoring element is covalently bonded to the particle.

The present invention relates to a diagnostic microarray plate comprising liposome nanoparticle for detecting the presence of specific metabolites in biofluids. The lipid nanoparticle comprises minimum one base or particles attached to a plate and/or embedded in said base; at least one element, such as an aptamer or nanobody needed to generate a color change in the lipid nanoparticle that is detectable by absorbance or fluorescence spectroscopy. The elements generate a change in the lipid nanoparticle on contact with a metabolite in biofluid.

Often metabolites found in biofluids are traceable in sufficient amounts to allow for a diagnostic system.

The lipid nanoparticle system enables persons trained in diagnostics to detect a set of metabolites on a single microarray plate. If the metabolite is present, a visible colorimetric change using absorbance or fluorescence spectroscopy can be used to determine the concentration of the said metabolite of the subject, which can provide an indication of health.

The present invention also addresses the challenge of screening metabolites from a single subject. Current systems typically use multiple systems that can lead to cross contamination, process difficulties and are time consuming.

The metabolite inducing colorimetric change is caused by a physical molecular change on the surface of the lipid nanoparticle caused by the metabolite binding to the aptamer or nanobody

The diagnostic microarray plate with the different lipid nanoparticles offers a novel way to monitor a number of metabolites in a similar manner as a way to avoid multiple cumbersome ELISA (Enzyme-Linked Immunosorbent Assay) kits or use of multiple different liquid chromatography assays. The plate enables the analysis of biofluids from a single subject and the simultaneous detection and/or quantification of several metabolites.

The diagnostic microarray plate with an isosceles trapezoid shaped well can sediment the cells to the bottom surface and concentrate the lipid nanoparticles to the upper surface. This enables an optimal spectral absorbance or fluorescence using a spectroscopy device.

Table 1 shows metabolites from different metabolite classes and some exemplary aptamer sequences suitable for practicing an embodiment of the present invention.

**Table 1. Metabolites and matching aptamers**

| **Metabolite Class** | **Metabolite** | **Aptamer Sequence** |
|---|---|---|
| Hormone | Cortisol | CTCTCGGGACGACGCCCGCATGTTCCATGGAT AGTCTTGACTAGTCGTCCC |
| Hormone | Dehydroepiandrosterone (DHEA) | CTGCTCTCGGGACGTGGATTTTCCGCATACGA AGTTGTCCCGAG |
| Hormone | Estradiol (17-Beta-Estradiol) | GCCGTTTGGGCCCAAGTTCGGC |
| Hormone | Estriol | TAGGGAAGAGAAGGACATATGATACATATCC GAAGGGTCCTGACCGGAGGCTGACCGGAGTG GGAATTGACTAGTACATGACCACTTGAGG |
| Hormone | Estrone | GCTTCCAGCTTATTGAATTACACGCAGAGGGT AGCGGCTCTGCGCATTCAATTGCTGCGCGCTG AAGCGCGGAAGC |
| Hormone | Progesterone | TAGGGAAGAGAAGGACATATGATGTCTGAGG TACTCACTTCTCACGTACGTTTCCCTCCTGAAT |
| | | TGACTAGTACATGACCACTTGAGG |
| Hormone | Testosterone | TAGGGAAGAGAAGGACATATGATGTGCCGTG AATACAGGCCCTTCTCCGCTCCGCGTTCCGCTT TGACTAGTACATGACCACTTGA |
| Hormone | Thyroid T4 thyroxine | TAATACGACTCACTATAGGGAATTCGTCGACG GATCCGCCGTTGGTGTTCGGTCAGGCTTCCGT GGCAACGGGGCAAAATGGTAATCGCGGGGAA CCTGCAGGTCGACGCATGCGCCG |
| Vitamin | Vitamin A (retinol) Binding Protein | ATACCAGCTTATTCAATTACGGTGCGGAGGGG GAGGGTGGCGGGTTGTGTCGGTGTGGAGATAG TAAGTGCAATCT |
| Vitamin | Vitamin B1 (thiamine) | CCACUAGGGGUGCUUGUUGUGCUGAGAGAGG AAUAAUCCUUAACCCUUAUAACACCUGAUCU AGGUAAUACUAGCGAAGGAAGUGG |
| Vitamin | Vitamin B2 (riboflavin) | AACGACCAGGTGAGTACAGCTAATTCATTAGG GTATGTCGTT |
| Vitamin | Vitamin D (25-Hydroxyvitamin D) | AGCAGCACAGAGGTCATGGGGGTGTGACTTT GGTGTGCCTATGCGTGCTACGGAA |

## Claims

1. A diagnostic microarray plate having a multitude of areas or wells for identifying or quantifying multiple metabolites in a biofluid, comprising:
I. a plastic base material with liposomal nanoparticles (liposomes or lipid embedded or attached or added) on the multitude of areas or wells of said base material; and
II. at least one element is present in each of the relevant areas or wells of the plate comprising an aptamer or a nanobody that is covalently attached to said liposomal nanoparticles, wherein said aptamer or nanobody can bind to at least one metabolite potentially present in said biofluid; and
III. a reporter material comprising a polymer, such as polydiacetylene, with or without a lipid that upon contact with a specific metabolite from said biofluid can generate a colorimetric change that is visible by absorbance or fluorescence spectroscopy or a chemical that changes color through a redox reaction.

2. The diagnostic microarray plate according to claim 1, in which said aptamer or nanobody attached to a reporter material is a molecular assembly or molecule, which upon contact with the metabolite induces a color change observable by the naked human eye, absorbance spectrometry or fluorescence spectrometry.

3. The diagnostic microarray plate according to any of claims 1-2, wherein said multitude of areas or wells each comprises an element comprising an aptamer or nanobody such that different metabolites in a biofluid sample from a single subject can be detected or quantified.

4. The diagnostic microarray plate according to any of the preceding claims, wherein said metabolites are selected from the group consisting of hormones, vitamins, salts, sterols, and triglycerides.

5. The diagnostic microarray plate according to any of the preceding claims, wherein said diagnostic microarray plate is for detection or quantification of at least 5 specific hormones or vitamins.

6. The diagnostic microarray plate according to any of the preceding claims, wherein said diagnostic microarray plate is for detection or quantification of at least 5 metabolites among the following list: Cortisol, Dehydroepiandrosterone (DHEA), Estradiol (17-Beta-Estradiol), Estriol, Estrone, Progesterone, Testosterone, Thyroid T4 thyroxine, Vitamin A (retinol) Binding Protein, Vitamin B1 (thiamine), Vitamin B2 (riboflavin), and Vitamin D (25-Hydroxyvitamin D).

7. The diagnostic microarray plate according to any of the preceding claims, wherein said aptamers or nanobodies are all attached to the liposome nanoparticles on the plate such that the diagnostic microarray plate can be used multiple times after washing.

8. The diagnostic microarray plate according to any of the preceding claims, wherein said reporter material is selected from the group consisting of:
- a molecule with peroxidase activity viz. horseradish peroxide, ruthenium nanoparticles, ribozymes, DNAzyme that can convert colorimetric reporters 3,3',5,5'-tetramethylbenzidine (TMB), Hemin or Amplexa Red through a chemical reaction;
- a liposomal sensor comprising a polymerized polydiacetylene molecule, wherein the liposome displays a color change of blue to red (absorbance in wavelengths 450-740 nm) or displays a fluorescent change (emission 510-700 nm with excitation at 500 nm) in the presence of the target analyte; and
- a complementary sequence containing a colorimetric or fluorescent reporter, where the complementary sequence is displaced.

9. The diagnostic microarray plate according to any of the preceding claims, wherein said biofluid is saliva.

10. The diagnostic microarray plate according to any of the preceding claims, wherein said liposomal nanoparticles are attached to polydiacetylene embedded in said plastic base material.

11. The diagnostic microarray plate according to any of the preceding claims, wherein each of said multitude of areas or wells are duplicated to accommodate for a standard for calibration purpose.

12. The diagnostic microarray plate according to any of the preceding claims, wherein the well is an isosceles trapezoid shape to adsorb cells on the bottom surface.

13. Use of a diagnostic microarray plate as defined in any of claims 1-12 for signature pattern analysis of a biofluid sample from a single subject.

14. Use of a diagnostic microarray plate according to any of claims 1-12 for the detection or quantification of at least 5 hormones and vitamins selected from the group consisting of: Cortisol, Dehydroepiandrosterone (DHEA), Estradiol (17-Beta-Estradiol), Estriol, Estrone, Progesterone, Testosterone, Thyroid T4 thyroxine, Vitamin A (retinol) Binding Protein, Vitamin B1 (thiamine), Vitamin B2 (riboflavin), and Vitamin D (25-Hydroxyvitamin D).

15. Use of a diagnostic microarray plate according to any of claims 1-12 for the simultaneous detection or quantification of the following hormones and vitamins in a biofluid sample: Cortisol, Dehydroepiandrosterone (DHEA), Estradiol (17-Beta-Estradiol), Estriol, Estrone, Progesterone, Testosterone, Thyroid T4 thyroxine, Vitamin A (retinol) Binding Protein, Vitamin B1 (thiamine), Vitamin B2 (riboflavin), and Vitamin D (25-Hydroxyvitamin D).
